# DEMANDE DE BREVET EUROPEEN

(11) **EP 1 535 601 A1**
(43) Date de publication de la demande: **01.06.2005**
(21) Numéro de dépôt: 04291870.6
(22) Date de dépôt: 23.07.2004
(51) Int. Cl.: A61K 7/13

(54) **Utilisation en coloration de dérivés de ninhydrine comportant un ou plusieurs cycles condensés**

(30) Priorité: 25.07.2003 FR 0309176; 04.03.2004 FR 0402245
(71) Demandeur: L'OREAL, 75008 Paris (FR)
(72) Inventeur: Plos, Grégory, Tokyo 158-0097 (JP); Gourlaouen, Luc, 92600 Asnières (FR)
(74) Mandataire: Dossmann, Gérard

(57) **Abrégé**

L'invention concerne l'utilisation pour la coloration de matières kératiniques de composition contenant, dans un milieu approprié pour la teinture, au moins un composé de formule (I) 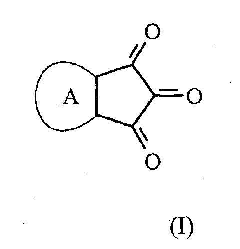 dans laquelle

A représente un polycycle aromatique condensé comportant au moins 12 chaînons, étant choisi, de préférence, parmi les groupes anthracènyle, tétracènyle, phénanthrènyle, azulènyle.

## Description

La présente invention concerne des compositions de coloration des matières kératiniques, en particulier des compositions de coloration capillaire contenant au moins un dérivé de ninhydrine comportant un ou plusieurs cycles condensés associé, de préférence, à un composé à fonction amine primaire ou secondaire ou à un composé à fonction méthylène activé, un procédé de coloration utilisant de telles compositions et un agent de coloration multi-composants servant à la mise en oeuvre d'un tel procédé.

Depuis longtemps, de nombreuses personnes cherchent à modifier la couleur de leur peau, de leurs cils ou de leurs cheveux et en particulier à masquer leurs cheveux blancs. Pour ce faire, plusieurs technologies ont été développées.

Il est connu de teindre les fibres kératiniques humaines, et en particulier les cheveux, avec des compositions tinctoriales contenant des précurseurs de colorants d'oxydation, appelés généralement bases d'oxydation. Ces bases d'oxydation sont des composés incolores ou faiblement colorés qui, associés à des produits oxydants, donnent naissance par un processus de condensation oxydative à des composés colorés. Ces colorants sont insolubles et sont piégés à l'intérieur de la fibre capillaire.

On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Les colorations obtenues présentent une bonne ténacité aux shampooings. Cependant, la réaction d'oxydation se fait à l'aide de produits oxydants tels l'eau oxygénée en milieu basique. Ces agents oxydants attaquent la kératine des cheveux dont les propriétés cosmétiques et mécaniques peuvent se dégrader fortement en cas de colorations répétées.

Il est aussi connu de teindre les fibres kératiniques humaines par une coloration directe consistant à appliquer sur les fibres kératiniques des colorants directs qui sont des molécules colorées et colorantes ayant une affinité pour les fibres. On peut citer à titre d'exemples de colorants directs utilisés classiquement les colorants nitrés, benzéniques, anthraquinoniques, nitropyridiniques, azoïques, azoïques cationiques, xanthéniques, acridiniques, aziniques ou de type triarylméthane ou des colorants naturels.

Les colorations obtenues ainsi sont, certes, très chromatiques et n'entraînent pas une dégradation chimique de la kératine, mais présentent l'inconvénient de n'être que temporaires ou semi-permanentes, c'est-à-dire de s'estomper au mieux après seulement 4 à 5 shampooings.

Il subsiste par conséquent un besoin de systèmes et de procédés de coloration qui permettent l'obtention de bonnes ténacités sans impliquer l'utilisation d'agents oxydants susceptibles de dégrader les matières kératiniques.

La demanderesse a découvert avec surprise que l'utilisation de dérivés de ninhydrine comportant un ou plusieurs cycles condensés décrits plus en détail ci-dessous permettait de colorer les matières kératiniques, et en particulier les cheveux, avec des ténacités équivalentes voire supérieures à celles obtenues par coloration d'oxydation et ceci en l'absence d'agents oxydants forts préservant ainsi parfaitement les matières kératiniques.

Les dérivés de ninhydrine mentionnés ci-dessus sont utilisés de préférence en combinaison avec des composés à hydrogène labile, tels que des amines primaires ou secondaires ou des composés à fonction méthylène activé.

Les colorations obtenues ainsi présentent de bonnes chromaticités et se distinguent en particulier par une excellente ténacité au lavage (plusieurs dizaines de shampooings).

L'invention a donc pour objet l'utilisation pour la coloration de matières kératiniques d'une composition contenant, dans un milieu approprié pour la teinture, au moins un composé de formule (I) ou sa forme tautomère : 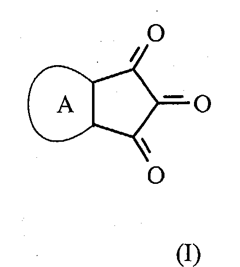 dans laquelle
A représente un polycycle aromatique condensé comportant au moins 12 chaînons.

Le groupement A est choisi, de préférence, parmi les groupes anthracènyle, tétracènyle, phénanthrènyle, azulènyle.

Le groupement A peut être substitué par des groupements halogéno, comme chloro, iodo, bromo et/ou fluoro, des groupements alkyle en C₁-C₆, des groupements hydroxy, des groupements alcoxy en C₁-C₆, des groupements amino, des groupements mono- ou di-(alkyle en C₁-C₆)amino, des groupements mono- ou di-hydroxy(alkyle en C₁-C₆)amino, des groupements tri(alkyle en C₁-C₆)ammonio, des groupements imidazolyle, des groupements pyridinyle, des groupements thio, des groupements (alkyle en C₁-C₆)thio, des groupements thio(alkyle en C₁-C₆), des groupements (alkyle en C₁-C₆)carbonyle, des groupements hydrogénocarbonyle, des groupements hydroxycarbonyle, des groupements (alcoxy en C₁-C₆)carbonyle, des groupements nitro, des groupements sulfonato, les groupes protonés correspondants tels que ammonio, imidazolio, et/ou pyridinio.

De telles compositions sont en particulier utiles pour la teinture des fibres kératiniques, notamment les cheveux.

Les composés de formule (I) englobent, bien entendu, également les sels d'addition d'acides et les sels d'addition de bases correspondants.

Les dérivés de ninhydrine de formule (I) ci-dessus sont utilisés dans la présente invention dans un milieu cosmétiquement acceptable contenant généralement une fraction importante d'eau. Lorsqu'ils sont dissous dans un tel milieu aqueux, les dérivés de ninhydrine de formule (I) sont en équilibre d'hydratation avec la forme diol géminé (ou hydrate de carbonyle) correspondant à la formule (I)bis suivante :

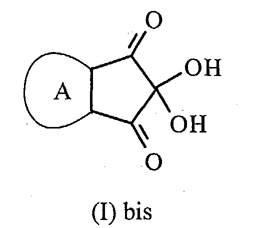

Lorsqu'il est question, dans la présente demande, de dérivés de ninhydrine de formule (I) ceux-ci englobent par conséquent toujours non seulement les composés de formule (I) mais également les formes hydratées correspondantes de formule (I)bis.

Des exemples préférés de dérivés de ninhydrine utilisables conformément à la présente invention pour la coloration des fibres capillaires sont les suivants :

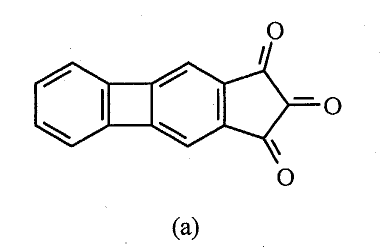

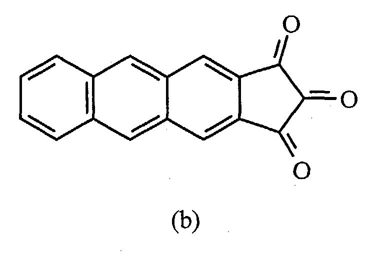

Les dérivés de ninhydrine utilisés dans la présente invention sont connus. La synthèse des dérivés de ninhydrine (a) et (b) ci-dessus est décrite dans les publications suivantes :
(a): Buckland P. R., Mc Omie J. F. W., Biphenylenes XXIX synthesis of cyclobuta[b]biphenylene- 1 -carboxylic acid and of cyclopenteno[b]biphenylene-1,2,3-trione, Tetrahedron, 1977, 33, 1797-1801.
(b) Hallman J.L., Bartsch R. A., Synthesis of naphtho[flninhydrin, J. Org. Chem. 1991, 56, 6243-6245

Conformément à la présente invention, les dérivés de ninhydrine de formule (I) décrits ci-dessus peuvent être utilisés seuls pour la coloration des matières kératiniques. En effet, ces composés sont capables de générer des molécules colorées avec les fonctions amine de la kératine (réaction colorée).

On peut aussi utiliser conjointement des composés de formule (I) avec au moins un activateur qui permet de modifier la cinétique de réaction du composé ninhydrine avec la matière kératinique. Un tel activateur peut être un agent oxydant, un agent réducteur, des acides de brönsted, un catalyseur métallique tel que les catalyseurs à base de métal de transition tel que le fer, le platine, le palladium, des protéines notamment des enzymes, des composés modifiant la force ionique du milieu tels que des sels de NaCl, des composés à hydrogène labile choisi parmi ceux comportant une fonction amine primaire ou secondaire et ceux comportant une fonction méthylène activé. On peut, bien entendu, utiliser également un mélange de tels composés.

Les composés à fonction amine primaire ou amine secondaire sont de préférence des amines aromatiques.

On peut citer à titre d'exemples de telles amines aromatiques la N,N-diméthyl-p-phénylènediamine, la N,N-diéthyl-p-phénylènediamine, la N-(2-hydroxyéthyl)-N-éthyl-p-phénylènediamine, la N,N-bis-(2-hydroxyéthyl)-p-phénylènediamine, la N-(2-méthoxyéthyl)-p-phénylènediamine, la 2,3-, 2,4- ou 2,5-dichloro-p-phénylène-diamine, la 2-chloro-p-phénylènediamine, le dibromhydrate de 2,5-dihydroxy-4-morpholinoaniline, le 2-, 3- ou 4-aminophénol, le 2-aminométhyl-4-aminophénol, le 2-hydroxyméthyl-4-aminophénol, l'ortho-phénylènediamine, la p-phénylènediamine, l'ortho-toluènediamine, le 2,5-diaminotoluène, le 2,5-diaminophénol, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, la 4-méthylaminoaniline, la 3-amino-4-(2'-hydroxyéthyloxy)aniline, la 3,4-méthylènediaminoaniline, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 6-méthyl-3-amino-2-chlorophénol, le 2-méthyl-5-amino-4-chlorophénol, le 3,4-méthylènedioxyphénol, le 5-(2-hydroxyéthylamino)-4-méthoxy-2-méthylphénol, le 4-amino-2-hydroxyméthylphénol, le 1,3-diamino-2,4-diméthoxybenzène, l'acide 2-, 3-, 4-aminobenzoïque, l'acide 2-amino-, 3-amino ou 4-aminophénylacétique, l'acide 2,3-, 2,4-, 2,5-, 3,4- ou 3,5-diaminobenzoïque, l'acide 4-amino- ou 5-amino-salicylique, l'acide 3-amino-4-hydroxybenzoïque, l'acide 4-amino-3-hydroxybenzoïque, l'acide 2-amino-, 3-amino ou 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, le 2,4,6-triaminorésorcinol, le 4,5-diaminopyrochatécol, le 4,6-diaminopyrogallol, le 3,5-diamino-4-hydroxypyrochatécol, et les anilines aromatiques et phénols aromatiques comportant un autre résidu aromatique, correspondant à la formule (II) 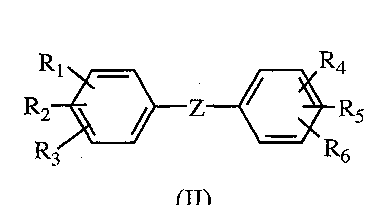 dans laquelle
R¹ représente un groupe hydroxy ou amino éventuellement substitués par un groupe alkyle en C₁₋₄, hydroxyalkyle en C₁₋₄ ou (alcoxy en C₁₋₄)-(alkyle en C₁₋₄),
R², R³, R⁴, R⁵ et R⁶ représentent chacun indépendamment un atome d'hydrogène, un groupe hydroxy ou un groupe amino, éventuellement substitués par un groupe alkyle en C₁₋₄, hydroxyalkyle en C₁₋₄ ou (alcoxy en C₁₋₄)-(alkyle en C₁₋₄), ou un groupe acide carboxylique ou sulfonique,
Z représente une liaison directe, une chaîne hydrocarbonée en C₁₋₄, saturée ou insaturée, éventuellement hydroxylée, un groupe carbonyle, sulfonyle ou imino, un atome d'oxygène ou de soufre, ou un groupe de formule Q-(CH₂-P-CH₂-Q')ₒ où P représente une liaison directe ou un groupe -CH₂- ou -CHOH-, Q et Q' représentent, indépendamment l'un de l'autre, un atome d'oxygène, un groupe NR⁷ où R⁷ représente un atome d'hydrogène, un groupe alkyle en C₁₋₄ ou hydroxyalkyle en C₁₋₄, ou un groupe O-(CH₂)ₚNH ou NH-(CH2)p'-O où p et p' valent 2 ou 3 et o est un nombre entre 1 et 4.

Les amines primaires ou secondaires non-aromatiques sont par exemple le 2-aminoéthanol, la 2-méthoxyéthylamine, la 2-éthoxyéthylamine, le 2-(2-aminoéthoxy)-éthanol, le 2- ou 3-aminopropanol, la 2,3-dihydroxypropylamine, la 4-hydroxypropylamine, le 2-aminopropane-1,3-diol, le 2-amino-2-méthylpropanol, le 2-amino-2-méthylpropane-1,3-diol, le 2-amino-2-hydroxyméthylpropane-1,3-diol, la tétrahydropentylamine, les pentahydroxyhexylamines telles que la glucamine, la D-glucosamine, la D-galactosamine, le 1,2-diaminoéthane, le 1,2- ou 1,3-diaminopropane, le 1,3-diamino-2-propanol, la 2-(2-amnoéthylamino)-éthylamine, le 2-(2-aminoéthylamino)éthanol, la 3-(2-aminoéthylamino)-propylamine et le 3-(2-aminoéthylamino)propanol.

Les composés à fonction méthylène activé sont choisis par exemple parmi les suivants : l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylène-indoline, l'iodure de 2,3-diméthylbenzothiazolium, le p-toluènesulfonate de 2,3-diméthyl-benzothiazolium, la rhodanine, l'acide rhodanine-3-acétique, l'iodure de 1-éthyl-2-quinaldinium, l'iodure de 1-méthyl-2-quinaldinium, l'acide barbiturique, l'acide thiobarbiturique, l'acide 1,3-diméthylthiobarbiturique, l'acide diéthylthiobarbiturique, l'oxindole, l'acétate de 3-indoxyle, la coumarone et la 1-méthyl-3-phényl-2-pyrazolinone.

Ces amines primaires et secondaires et ces composés à fonctions méthylène activé ainsi que d'autres composés à hydrogène labile sont décrits également dans les demandes de brevet DE 43 17 855, DE 197 17 222, DE 198 45 481 et DE 197 45 355 où ils sont utilisés pour la coloration des fibres kératiniques en combinaison avec des composés différents des dérivés de ninhydrine de formule (I).

Lorsque les dérivés de ninhydrine de formule (I) sont utilisés en combinaison avec une amine primaire ou secondaire ou avec un composé à fonction méthylène activé, il est nécessaire de conserver ces différents réactifs séparément pour éviter une réaction colorée prématurée. La mise en contact des réactifs ne se fait alors qu'immédiatement avant application sur les cheveux, par mélange extemporané de deux compositions contenant respectivement les dérivés de ninhydrine et les composés à hydrogène labile. La mise en contact des réactifs peut se faire aussi directement sur le cheveu par application successive des différents réactifs.

L'invention a par conséquent également pour objet un agent de coloration multi-composants comportant
- en tant que premier composant, une composition (a) contenant au moins un dérivé de ninhydrine de formule (I), et
- en tant que deuxième composant, une composition (b) contenant au moins un composé à fonction amine primaire ou secondaire ou au moins un composé à fonction méthylène activé, tels que décrits ci-dessus.

Cet agent de coloration multi-composants se présente de préférence sous forme d'un kit multi-compartiments, avec au moins un premier compartiment contenant le premier composant (composition (a)) et au moins un deuxième compartiment contenant le deuxième composant (composition (b)).

L'invention a également pour objet une composition cosmétique tinctoriale contenant au moins un dérivé de ninhydrine de formule (I) et au moins un principe actif cosmétique.

Les principes actifs cosmétiques présents dans les compositions cosmétiques de la présente invention sont choisis par exemple parmi les vitamines, les saccharides, les oligosaccharides, les polysaccharides hydrolysés ou non, modifiés ou non, les acides aminés, les oligopeptides, les peptides, les protéines hydrolysées ou non, modifiées ou non, les polyacides aminés, les enzymes, les acides et alcools gras ramifiés ou non, les cires animales, végétales ou minérales, les céramides et les pseudo-céramides, les acides organiques hydroxylés, les filtres UV, les agents anti-oxydants et les agents anti-radicaux libres, les agents chélatants, les agents antipelliculaires, les agents régulateurs de séborrhée, les agents apaisants, les agents tensioactifs cationiques, anioniques, non-ioniques ou amphotères, les polymères cationiques, anioniques, neutres ou amphotères, les silicones organomodifiées ou non, les huiles minérales, végétales ou animales, les polyisobutènes et poly(α-oléfines), les esters gras, les polymères anioniques sous forme dissoute ou dispersée, les polymères non-ioniques sous forme dissoute ou dispersés, les agents réducteurs, les solvants, les colorants capillaires tels que les colorants directs ou les précurseurs de coloration d'oxydation (bases et/ou coupleurs) différents des composés à fonction amine primaire ou secondaire revendiqués, les agents oxydants tels que le peroxyde d'hydrogène éventuellement associé à des persels, les pigments et leurs mélanges.

Le principe actif cosmétique est présent de préférence à raison de 0,001 à 50 % en poids, en particulier de 0,01 à 20 % en poids, et idéalement à raison de 0,1 à 10 % en poids, rapporté au poids total de la composition cosmétique.

Dans un mode de réalisation préféré de la composition cosmétique tinctoriale selon l'invention, le principe actif cosmétique est un agent tensioactif et/ou un agent polymèrique (polymère), ces agents pouvant être de nature non-ionique, cationique, anionique ou amphotère.

Il ressort de ce qui précède que les compositions de coloration capillaire utilisées dans la présente invention sont stables au stockage lorsqu'elles contiennent, comme seuls réactifs, des dérivés de ninhydrine de formule (I). Mais lorsqu'elles contiennent à la fois des dérivés de ninhydrine de formule (I) et des composés à hydrogène labile tels que des amines primaires ou secondaires ou des composés à fonction méthylène activé, ces compositions doivent être utilisées immédiatement après mélange de la composition contenant les dérivés de ninhydrine de formule (I) avec celle contenant le ou les composés à hydrogène labile.

Ces compositions de coloration prêtes à l'emploi, qu'elles soient stables au stockage ou préparées immédiatement avant emploi, ont de préférence un pH compris entre 2 et 12, et en particulier entre 3 et 11.

Leur teneur en dérivés de ninhydrine de formule (I) est de préférence comprise entre 0,0001 % et 30 % en poids.

Les composés à hydrogène labile utilisés en combinaison avec les dérivés de ninhydrine de formule (I) sont de préférence présents à raison de 0,0001 à 30% en poids par rapport au poids total de la composition.

La présente invention a en outre pour objet un procédé de coloration capillaire comprenant l'application, sur les cheveux, d'une composition de coloration capillaire prête à l'emploi telle que décrite ci-dessus. Cette composition est laissée en contact avec les fibres capillaires pendant un temps suffisant pour l'obtention de la coloration désirée. Ce temps de pose est généralement compris entre 5 minutes et 1 heure, et en particulier entre 15 et 30 minutes. La réaction colorée entre les dérivés de ninhydrine et les fonctions amine de la kératine ou les composés à hydrogène labile éventuellement présents, peut être accélérée par chauffage des cheveux imprégnées avec la composition de coloration. La température de chauffage ne dépasse de préférence pas 80 °C, et est en particulier inférieure ou égale à 60 °C.

Après obtention de la coloration souhaitée, les cheveux sont rincés et lavés.

Lorsqu'on utilise des composés à hydrogène labile tels que des amines primaires ou secondaires ou des composés à fonction méthylène activé, l'application des réactifs participant à la réaction colorée peut également se faire en deux temps, autrement dit on peut appliquer successivement deux compositions différentes contenant respectivement au moins un dérivé de ninhydrine de formule (I) et au moins un composé comportant une fonction amine primaire ou secondaire ou une fonction méthylène activé.

La présente invention a par conséquent également pour objet un procédé de coloration en deux temps comprenant le fait d'appliquer sur les cheveux l'une après l'autre, dans n'importe quel ordre, une composition (a) et une composition (b) telles que définies ci-dessus pour l'agent de coloration multi-composants.

Cette application séparée des deux compositions réactives présente l'avantage d'éviter la manipulation de compositions colorées et diminue ainsi les risques de souillure de matériaux tels que les vêtements.

La demanderesse a constaté que l'on obtenait également des colorations capillaires satisfaisantes lorsqu'une étape de rinçage intermédiaire était insérée entre l'application de la première composition et l'application de la deuxième composition.

De manière analogue à celle décrite ci-dessus, les cheveux imprégnés de composition (a) et/ou (b) peuvent être chauffés, de préférence jusqu'à une température de 80 °C, et en particulier jusqu'à une température ne dépassant pas 60 °C, un tel chauffage permettant d'accélérer la réaction colorée et de raccourcir le temps de pose.

### Exemple :

On prépare la composition suivante :

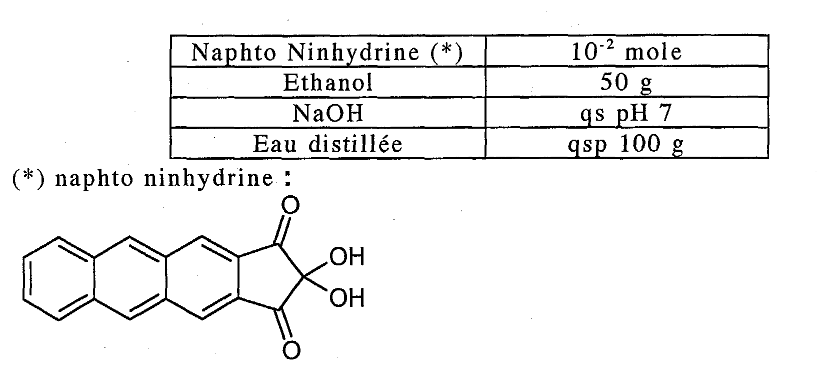

On applique cette composition sur deux mèches de cheveux naturels et permanentés à 90 % de cheveux blancs, de 1 g chacune. Le rapport de bain est de 5, le temps de pose de 30 minutes et la température de 60 °C. A la fin du temps de pose, les mèches sont rincées, puis lavées avec un shampooing standard.

Les mèches obtenues sont colorées et ont un reflet doré.

## Revendications

1. Utilisation pour la coloration de matières kératiniques d'une composition contenant, dans un milieu approprié pour la teinture, au moins un composé de formule (I) 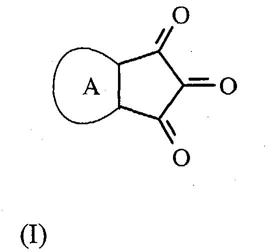 dans laquelle
A représente un polycycle aromatique condensé comportant au moins 12 chaînons.

2. Utilisation selon la revendication 1, **caractérisée par le fait que** le groupement A est choisi parmi les groupes anthracényle, tétracényle, phénanthrènyle, azulènyle.

3. Utilisation selon la revendication 1 ou 2, **caractérisée par le fait que** le groupement A est substitué par un ou plusieurs groupements choisis parmi des groupements halogéno, groupements alkyle en C₁-C₆, groupements hydroxy, groupements alcoxy en C₁-C₆, groupements amino, groupements imidazolyle, groupements pyridinyle, groupements mono- ou di-(alkyle en C₁-C₆)amino, groupements mono- ou di-hydroxy(alkyle en C₁-C₆)amino, groupements tri(alkyle en C₁-C₆)ammonio, groupements thio, groupements (alkyle en C₁-C₆)thio, groupements thio(alkyl en C₁-C₆), groupements (alkyle en C₁-C₆)carbonyle, groupements hydrogénocarbonyle, groupements hydroxycarbonyle, groupements (alcoxy en C₁-C₆)carbonyle, groupements nitro, groupements sulfonato, les groupes protonés correspondants tels que ammonio,, imidazolio, et/ou pyridinio.

4. Utilisation selon l'une quelconque des revendications 1 à 3, **caractérisée par le fait qu'**elle comprend en outre au moins un activateur qui permet de modifier la cinétique de réaction du composé ninhydrine avec la matière kératinique.

5. Utilisation selon la revendication 4, **caractérisée par le fait que** l'activateur est choisi parmi un agent oxydant, un agent réducteur, des acides de brönsted, un catalyseur métallique, des protéines, des composés modifiant la force ionique du milieu, des composés à hydrogène labile choisi parmi ceux comportant une fonction amine primaire ou secondaire et ceux comportant une fonction méthylène activé ou un mélange de ceux-ci.

6. Utilisation selon la revendication 5, **caractérisée par le fait que** le composé comportant une fonction amine primaire ou secondaire est une amine aromatique choisie parmi la N,N-diméthyl-p-phénylènediamine, la N,N-diéthyl-p-phénylènediamine, la N-(2-hydroxy-éthyl)-N-éthyl-p-phénylènediamine, la N,N-bis-(2-hydroxyéthyl)-p-phénylène-diamine, la N-(2-méthoxyéthyl)-p-phénylènediamine, la 2,3-, 2,4- ou 2,5-dichloro-p-phénylène-diamine, la 2-chloro-p-phénylènediamine, le dibromhydrate de 2,5-dihydroxy-4-morpholinoaniline, le 2-, 3- ou 4-aminophénol, le 2-aminométhyl-4-aminophénol, le 2-hydroxyméthyl-4-aminophénol, l'ortho-phénylènediamine, la p-phénylènediamine, l'ortho-toluènediamine, le 2,5-diaminotoluène, le 2,5-diaminophénol, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, la 4-méthylaminoaniline, la 3-amino-4-(2'-hydroxyéthyloxy)aniline, la 3,4-méthylènediaminoaniline, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 6-méthyl-3-amino-2-chlorophénol, le 2-méthyl-5-amino-4-chlorophénol, le 3,4-méthylènedioxyphénol, le 5-(2-hydroxyéthylamino)-4-méthoxy-2-méthylphénol, le 4-amino-2-hydroxyméthylphénol, le 1,3-diamino-2,4-diméthoxybenzène, l'acide 2-, 3-, 4-aminobenzoïque, l'acide 2-amino-, 3-amino ou 4-aminophénylacétique, l'acide 2,3-, 2,4-, 2,5-, 3,4- ou 3,5-diaminobenzoïque, l'acide 4-amino- ou 5-amino-salicylique, l'acide 3-amino-4-hydroxybenzoïque, l'acide 4-amino-3-hydroxybenzoïque, l'acide 2-amino-, 3-amino ou 4-aminobenzènesulfonique, l'acide 3-amino-4-hydroxybenzènesulfonique, l'acide 4-amino-3-hydroxynaphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-triaminobenzène, le 1,2,4-triaminobenzène, le 1,2,4,5-tétraaminobenzène, le 2,4,5-triaminophénol, le pentaaminobenzène, l'hexaaminobenzène, le 2,4,6-triaminorésorcinol, le 4,5-diaminopyrochatécol, le 4,6-diaminopyrogallol, le 3,5-diamino-4-hydroxypyrochatécol, et les anilines aromatiques et phénols aromatiques comportant un autre résidu aromatique, correspondant à la formule (II) suivante 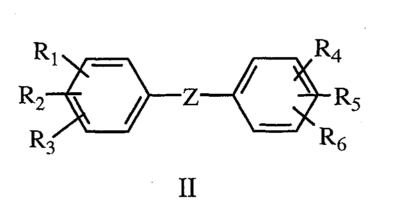 dans laquelle
R¹ représente un groupe hydroxy ou amino éventuellement substitués par un groupe alkyle en C₁₋₄, hydroxyalkyle en C₁₋₄ ou (alcoxy en C₁₋₄)-(alkyle en C₁₋₄),
R², R³, R⁴, R⁵ et R⁶ représentent chacun indépendamment un atome d'hydrogène, un groupe hydroxy ou un groupe amino, éventuellement substitués par un groupe alkyle en C₁₋₄, hydroxyalkyle en C₁₋₄ ou (alcoxy en C₁₋₄)-(alkyle en C₁₋₄), ou un groupe acide carboxylique ou sulfonique,
Z représente une liaison directe, une chaîne hydrocarbonée en C₁₋₄, saturée ou insaturée, éventuellement hydroxylée, un groupe carbonyle, sulfonyle ou imino, un atome d'oxygène ou de soufre, ou un groupe de formule Q-(CH₂-P-CH₂-Q')ₒ où P représente une liaison directe ou un groupe -CH₂- ou -CHOH-, Q et Q' représentent, indépendamment l'un de l'autre, un atome d'oxygène, un groupe NR⁷ où R⁷ représente un atome d'hydrogène, un groupe alkyle en C₁₋₄ ou hydroxyalkyle en C₁₋₄, ou un groupe O-(CH₂)ₚNH ou NH-(CH₂)_{p'}-O où p et p' valent 2 ou 3 et o est un nombre entre 1 et 4
ou une amine aliphatique choisie parmi le 2-aminoéthanol, la 2-méthoxyéthylamine, la 2-éthoxyéthylamine, le 2-(2-aminoéthoxy)-éthanol, le 2- ou 3-aminopropanol, la 2,3-dihydroxypropylamine, la 4-hydroxypropylamine, le 2-aminopropane-1,3-diol, le 2-amino-2-méthylpropanol, le 2-amino-2-méthylpropane-1,3-diol, le 2-amino-2-hydroxyméthylpropane-1,3-diol, la tétrahydropentylamine, les pentahydroxyhexylamines telles que la glucamine, la D-glucosamine, la D-galactosamine, le 1,2-diaminoéthane, le 1,2- ou 1,3-diaminopropane, le 1,3-diamino-2-propanol, la 2-(2-amnoéthylamino)-éthylamine, le 2-(2-aminoéthylamino)éthanol, la 3-(2-aminoéthylamino)-propylamine et le 3-(2-aminoéthylamino)propanol.

7. Utilisation selon la revendication 5, **caractérisée par le fait que** le composé à fonction méthylène activé est choisi parmi l'iodure de 1,2,3,3-tétraméthyl-3H-indolium, le p-toluènesulfonate de 1,2,3,3-tétraméthyl-3H-indolium, le méthanesulfonate de 1,2,3,3-tétraméthyl-3H-indolium, la 1,3,3-triméthyl-2-méthylène-indoline, l'iodure de 2,3-diméthylbenzothiazolium, le p-toluènesulfonate de 2,3-diméthyl-benzothiazolium, la rhodanine, l'acide rhodanine-acétique, l'iodure de 1-éthyl-2-quinaldinium, l'iodure de 1-méthyl-2-quinaldinium, l'acide barbiturique, l'acide thiobarbiturique, l'acide 1,3-diméthylthiobarbiturique, l'acide diéthylthiobarbitique, l'oxindole, l'acétate de 3-indoxyle, la coumarone et la 1-méthyl-3-phényl-2-pyrazinone.

8. Utilisation selon l'une quelconque des revendications précédentes, **caractérisée par le fait que** la composition a un pH compris entre 2 et 12, de préférence entre 3 et 11.

9. Utilisation selon l'une quelconque des revendications précédentes, **caractérisée par le fait que** la concentration du composé de formule (I) est comprise entre 0,0001 % et 30 %, rapporté au poids total de la composition.

10. Utilisation selon l'une quelconque des revendications précédentes, **caractérisée par le fait que** la concentration du composé à fonction méthylène activé ou du composé comportant une fonction amine primaire ou secondaire est comprise entre 0,0001% et 30% rapporté au poids total de la composition.

11. Composition cosmétique tinctoriale comprenant, dans un milieu approprié pour la teinture des fibres kératiniques, au moins un dérivé de ninhydrine de formule (I) tel que défini dans les revendications 1 à 3 et au moins un agent tensioactif et/ou un agent polymérique de nature non ionique, cationique, anionique ou amphotère.

12. Composition cosmétique prête à l'emploi contenant au moins un dérivé de ninhydrine de formule (I) tel que défini dans les revendications 1 à 3 et au moins un composé contenant une fonction amine primaire ou secondaire ou au moins un composé à fonction méthylène activé ou un mélange de ceux-ci.

13. Agent de coloration multi-composants comportant
• en tant que premier composant, une composition (a) contenant au moins un composé de formule (I) tel que défini dans les revendications 1 à 3, et
• en tant que deuxième composant, une composition (b) contenant au moins un activateur tel que défini dans l'une quelconque des revendications 4 à 7.

14. Agent de coloration selon la revendication 13, **caractérisé par le fait qu'**il se présente sous forme d'un kit multi-compartiments, avec au moins un premier compartiment contenant la composition (a) et au moins un deuxième compartiment contenant la composition (b).

15. Procédé de coloration capillaire comprenant l'application, sur les cheveux, d'une composition de coloration capillaire telle que définie dans les revendications 1 à 10, un temps de pose suffisant pour permettre l'obtention de la coloration souhaitée, puis le rinçage et le lavage des cheveux.

16. Procédé de coloration capillaire selon la revendication 15, **caractérisé par le fait qu'**il comprend le chauffage des cheveux imprégnés de composition de coloration capillaire jusqu'à une température de 80 °C, de préférence de 60 °C.

17. Procédé de coloration capillaire comprenant le fait d'appliquer sur les cheveux l'une après l'autre, dans n'importe quel ordre, une composition (a) et une composition (b) telles que définies dans la revendication 13.

18. Procédé de coloration capillaire selon la revendication 17, **caractérisé par le fait qu'**une étape de rinçage intermédiaire est insérée entre l'application de la première composition et l'application de la deuxième composition.

19. Procédé de coloration capillaire selon l'une quelconque des revendications 17 ou 18, comprenant le chauffage des cheveux imprégnés avec la composition (a) et/ou (b) jusqu'à une température de 80 °C, de préférence de 60 °C.
